# EUROPEAN PATENT APPLICATION

(11) **EP 4 717 222 A1**
(43) Date of publication of application: **01.04.2026**
(21) Application number: 25204786.5
(22) Date of filing: 25.09.2025
(51) Int. Cl.: A61B 34/20

(54) **MULTIPLE VERTEBRA TRACKING**

(30) Priority: 25.09.2024 US 202463698756 P; 25.09.2024 EP 24202708
(71) Applicant: Fiagon GmbH, 16761 Hennigsdorf (DE)
(72) Inventor: HEDERMANN, Robert, 10409 Berlin (DE)
(74) Representative: Eisenführ Speiser

(57) **Abstract**

The invention refers to a system and method of image guided navigation. The system comprises:
a model generating unit (102) that is configured for generating a 3D model of a mammal form preoperatively obtained tomographic image data,
a segmentation unit (104) that is configured for segmenting the 3D model for obtaining multiple 3D model segments wherein at least some 3D model segments represent rigid body parts, in particular integral bone parts such as individual vertebra bodies or bone fragments, that may move with respect to each other,
a coordinate transformation unit (106) that is configured for generating individual coordinate system transformations using a navigation system for the image guided navigation by way of registration for at least some of the 3D model segments
a tracking unit (108) that is configured for intraoperatively tracking the position and orientation of a plurality of individual body parts of the mammal by means of the navigation system,
a visualization unit (110) that is configured for adapting a visualization of the 3D model in accordance with the respectively tracked positions and orientations of the individual body parts so as to visualize the individual body parts with their actual position and orientation relative to each other in the visualization of the 3D model, and
a display (112) that is connected to the visualization unit and being configured for displaying the adapted visualization of the 3D model.

## Description

### Background

The invention relates to a method and a system for an image guided instrument navigation system.

For an image guided instrument navigation, a trackable instrument is tracked by means of an instrument tracking system and the position and orientation of the tracked instrument is viewed on a display together with a representation of an anatomical structure of an object or a person. The representation of the anatomical structure is generated from tomographic image data of the object or person that is examined or treated by means of the tracked instrument.

The visualization of the tracked instrument together with the representation of the anatomical structure of an object or a person on the display may help a user to steer and manipulate the instrument as needed.

### Summary

In a first aspect, the invention provides a method of image guided navigation, comprising the steps of (a) generating a 3D model of a mammal form preoperatively obtained tomographic image data, (b) segmenting the 3D model for obtaining multiple 3D model segments wherein at least some 3D model segments represent rigid body parts, in particular integral bone parts such as individual vertebra bodies or bone fragments, that may move with respect to each other, (c) generating individual coordinate system transformations using a navigation system for the image guided navigation by way of registration for at least some of the 3D model segments, (d) intraoperatively tracking the position and orientation of a plurality of individual body parts of the mammal by means of the navigation system, (e) adapting a visualization of the 3D model in accordance with the respectively tracked positions and orientations of the individual body parts so as to visualize the individual body parts with their actual position and orientation relative to each other in the visualization of the 3D model, wherein the adaptation of the visualization of the 3D model comprises applying the individual coordinate system transformations to the respective tracked positions and orientations of the individual body parts, and (f) displaying the adapted visualization of the 3D model.

In various embodiments, the navigation system is an electromagnetic navigation system.

In some embodiments, the generation of individual coordinate system transformations using a navigation system for the image guided navigation by way of registration comprises intraoperative recording of image data by way of intraoperative 3D imaging or fluoroscopy imaging.

In some embodiments, the methods of the invention further comprise segmentation of the intraoperatively recorded images as represented by the intraoperatively recorded image data and performing registration between 3D model segments of the 3D model and corresponding segments of the intraoperatively recorded images.

In various embodiments, the methods of the invention further comprise attaching patient localizers to a plurality of rigid body parts, in particular to a plurality of vertebra bodies.

In some embodiments, the patient localizers are Kirschner-wires or Jamshidi-needles that each comprise a localizer coil.

In a further aspect, the invention provides a system for image guided navigation. The system comprises a model generating unit that is configured for generating a 3D model of a mammal form preoperatively obtained tomographic image data. The system further comprises a segmentation unit that is configured for segmenting the 3D model for obtaining multiple 3D model segments wherein at least some 3D model segments represent rigid body parts, in particular integral bone parts such as individual vertebra bodies or bone fragments, that may move with respect to each other. The system further comprises a coordinate transformation unit that is configured for generating individual coordinate system transformations using a navigation system for the image guided navigation by way of registration for at least some of the 3D model segments. The system further comprises a tracking unit that is configured for intraoperatively tracking the position and orientation of a plurality of individual body parts of the mammal by means of the navigation system. The system further comprises a visualization unit that is configured for adapting a visualization of the 3D model in accordance with the respectively tracked positions and orientations of the individual body parts so as to visualize the individual body parts with their actual position and orientation relative to each other in the visualization of the 3D model. The adaptation of the visualization of the 3D model as performed by the visualization unit comprises applying the individual coordinate system transformations to the respective tracked positions and orientations of the individual body parts. The system further comprises a display being connected to the visualization unit and being configured for displaying the adapted visualization of the 3D model.

In a further aspect, the invention provides a method of operating the system f the invention. The method comprises the steps of:
(a) generating a 3D model of a mammal form preoperatively obtained tomographic image data by means of the model generating unit,
(b) segmenting - by the segmenting unit - the 3D model for obtaining multiple 3D model segments wherein at least some 3D model segments represent rigid body parts, in particular integral bone parts such as individual vertebra bodies or bone fragments, that may move with respect to each other,
(c) generating - by the coordinate transformation unit - individual coordinate system transformations using a navigation system for the image guided navigation by way of registration for at least some of the 3D model segments,
(d) intraoperatively tracking the position and orientation of a plurality of individual body parts of the mammal by means of the navigation system by means of the tracking unit,
(e) adapting - by the visualization unit - a visualization of the 3D model in accordance with the respectively tracked positions and orientations of the individual body parts so as to visualize the individual body parts with their actual position and orientation relative to each other in the visualization of the 3D model, wherein the adaptation of the visualization of the 3D model comprises applying the individual coordinate system transformations to the respective tracked positions and orientations of the individual body parts, and
(f) displaying - by the display - the adapted visualization of the 3D model.

### Brief Description of the Drawings

The invention and further aspects shall now be described by way of exemplary embodiments with reference to the figures.

In the figures:
- Fig. 1A and B:: illustrate how the shape of the spine depends on the posture of the patient;
- Fig. 1C:: illustrates the decreasing accuracy of registration if a single vertebra body is tracked;
- Fig. 2:: further illustrates the mismatch resulting from different shapes of the spine caused by different postures of a patient;
- Fig. 3A and B:: illustrate the segmentation of intraoperatively obtained images of a spine;
- Fig. 4A:: shows an intraoperatively obtained gyroscopic image before surgery;
- Fig. 4B:: shows an intraoperatively obtained gyroscopic image after surgery;
- Fig. 5A and B:: illustrate how an individual registration of rigid body parts, in particular an individual registration of vertebra bodies may result in adapted visualization reflecting the actual position and orientation of the individual rigid body parts;
- Fig. 6:: illustrates how individual vertebra bodies - or individual rigid body parts in general - may be equipped individual patient localizers;
- Fig. 7A and B:: illustrate how the individual registration of individual segments can reflect intraoperative movement of predefined areas of image data;
- Fig. 8:: illustrates a system 100 for image guided navigation
- Fig. 9:: is a schematic representation of o method for operating the system of figure 8.

### Detailed Description

The instrument tracking system can be an electromagnetic (EM) tracking system that comprises at least one electromagnetic field generator that generates an electromagnetic field (EM field) in a tracking space. The electromagnetic field varies over time and position.

The trackable instrument may comprise electromagnetic field sensors (EM sensors) comprising at least one, or, alternatively, at least two sensor coils that can sense the electro-magnetic field. Because the electromagnetic field is controlled to vary over time and position, electric signals captured by the sensor coils of an EM sensor can be evaluated to thus determine the position of the EM sensor - and thus the instrument - in the tracking space. The evaluation comprises measuring the voltage induced in each sensor coil. Thus, an EM sensor can provide an output signal (hereinafter also called sensor signal) that is a voltage signal that can be evaluated to find the position of the sensor inside the EM field, i.e. in the tracking space. An EM sensor with a single sensor coil can provide a sensor signal that allows determining of the position and the orientation of the EM sensor within the tracking space with respect to five degrees of freedom (5 DoF), i.e. the position in three dimensions and the orientation in two dimensions. For determining the position and the orientation of an EM sensor in all six degrees of freedom (the position in three dimensions and the orientation in three dimensions), two sensor coils are needed that are arranged at an angle with respect to one another. Typically, a 6 DoF EM sensor comprises two sensor coils each having a longitudinal axis wherein the two longitudinal axis are arranged perpendicular with respect to each other. A 6 DoF EM sensor can produce two sensor signals, i.e. one sensor signal per sensor coil.

The EM sensors comprising the sensor coils can be very small and thus can be attached to a variety of instruments, for instance surgical instruments. Thus surgical instruments can be tracked even inside a body without a line-of-sight between the sensors and the field generator.

The EM sensors comprising the sensor coils are connected to an evaluation unit that evaluates the electric signals captured by the sensor coils, for instance the voltage induced into the sensor coils by the electromagnetic field generated and emitted by the EM field generator. The EM field generator is also connected to and controlled by the evaluation unit.

In order to maintain a proper registration between the EM field and a patient for whom a tomographic 3D model may exist that can be used like a three-dimensional map of the patient, a patient localizer can be attached to a patient. The patient localizer comprises sensor coils and thus allows tracking of the position and orientation of the patient in the EM field. This enables determining a relative position of a tracked instrument relative to the (tracked) patient and in turn allows real-time visualization of the instrument in a 3D model of the patient.

EM navigation is particularly useful if a 3D model of a patient exists and the position and orientation of a tracked instrument (i.e. an instrument with an EM sensor) can be shown in the 3D model during treatment. As known in the art, a 3D model of a patient can be obtained from pre-operative images for instance by means of tomography, for instance using magnetic resonance imaging (MRI). To allow proper alignment of the coordinate system of a 3D model and a real object, for instance a living patient, registration is needed. The EM navigation (i.e. the EM tracking system) as capable to determine the position and orientation of localizers in a coordinate system of the EM navigation system. Registration allows the show the position and orientation of the localizers in real-time images of for instance a patient. Real time images may be obtained with different modalities, for instance a comput-ertomograph or an optical system such as an endoscope that can capture optical images. Likewise, the position and orientation of the localizers can be displayed in 3D images that are reconstructed prom the preoperatively acquired 3D data. In order to track the position and orientation of instruments, localizers are arranged on or in an instrumented to be tracked. Therefore it is possible to display an instrument's position and orientation in real time or reconstructed images. In particular, renderings (i.e. reconstructed 3D images) of an instrument can be super imposed onto reconstructed or real-time images of a patient's anatomy.

One way to achieve registration is photo registration. Photo registration prior to a surgery using electromagnetic navigation refers to the process of incorporating pre-operative images, typically in the form of computer tomography scans or magnetic resonance imaging scans, into a surgical navigation system that utilizes electromagnetic technology, for instance an EM tracking system.

The steps involved in the photo registration process typically are:
1. Acquisition of pre-operative Images:
   Before the surgery, a patient undergoes tomographic imaging using computer tomography or magnetic resonance imaging, which capture detailed images of the anatomy relevant to the planned surgery.
2. Creation of a digital 3D model:
   The acquired images are converted into a digital 3D model using specialized software. This model accurately represents the patient's anatomy and serves as a reference for the surgical navigation system.
3. Registration:
   The next step involves aligning the digital 3D model with the patient's actual anatomy. Electromagnetic sensors placed on the patient's body serve as reference points for the tracking system to precisely detect and track the patient's position and orientation. The electromagnetic sensors placed on the patient's body are typically part of a patient localizer that is attached to the patient.
4. EM tracking system calibration:
   The electromagnetic tracking system needs to be calibrated to establish a relationship between the position of the sensors and the corresponding location on the digital 3D model. This calibration allows the system to accurately overlay the digital model onto the patient's anatomy.
5. Overlaying the digital 3D model:
   After calibration of the EM tracking system, the digital 3D model is superimposed onto the patient's real-time anatomy. This allows the surgeon to visualize and track instruments for navigating tracked instruments through the patient's body during the surgery using the guidance provided by the registered images.

The step of EM tracking system calibration is a crucial step in the photo registration process that ensures accurate tracking and alignment of the EM tracking system with the patient's anatomy. The calibration step involves:
- Taking a stereoscopic or 3D image of the patient and the patient localizer with a stereoscopic or a 3D camera. The 3D camera may be a time-of-flight (ToF) camera.
- The image taken by the camera is processed to generate a point cloud. The point cloud is a dense collection of 3D data points that represent the patient's outer appearance in a 3D image together with the patient localizer.
- The point cloud generated from the 3D images taken by the camera is than aligned with the patient's anatomy as represented in the pre-operative, digital 3D model.
- Once the point cloud generated from the 3D images taken by the camera and the patient's anatomy as represented in the pre-operative 3D model are aligned, the relationship between the positions and orientations of the anatomic structures as represented in the 3D model and the EM tracking system's coordinate system can be established.
- The calibration process may involve additional steps to optimize the accuracy of the alignment. This may include fine-tuning the registration based on known anatomical landmarks or performing iterative adjustments to improve the system's accuracy. Once the calibration is completed, the accuracy and reliability of the EM tracking system are verified and validated. This may involve conducting calibration tests and quality control checks to ensure the system's performance meets the intended accuracy requirements.

Method and a systems for an image guided instrument navigation are inter alia known from US 10,568,713 B2, US 2010/0249506 A1, WO 2021/069745 and US 9,641,808 B2.

During an operation - for instance a surgery - , a patient may move and thus proper registration may be affected. In particular the vertebra of a spine are moving with respect to one another is the patient changes its posture. Therefore, it is not sufficient to track a patient's position by means of a patient localizer for maintaining proper registration of the individual vertebra.

So far, navigation accuracy is usually limited to body regions adjacent to a vertebra body that holds a patient localizer. Especially, when preoperative image data is used which is usually taken in supine position, while intraoperative patient positioning is usually in prone position. Even with intraoperative image data movement of the vertebra bodies relative to each other decreases the accuracy, since only one patient localizer is fixed to one vertebra body.

The invention includes the recognition that the use of preoperatively obtained image data and a 3D model derived therefrom as a basis for navigation does not necessarily reflect an actual 3D configuration of e.g. a person. This image data may differ from the actual situation in the operation room, due to patient positioning.

To mitigate this problem, a patient registration may be carried out intraoperatively, either with intraoperative 3D imaging or with Fluoroscopy imaging representing the correct alignment.

However, even if Intraoperative image data is used as a basis for navigation, this image data gets invalidated with intraoperative movement of the vertebra bodies, for instance after cage placement, spinal realignment) or the like.

To mitigate this problem, it is proposed to track the movement of single vertebra bodies intraoperatively and manipulate/augment image data to keep its validity throughout the surgical process.

In particular, it is proposed to
- equip multiple vertebra bodies with patient localizers,
- segment the image data into vertebra body areas wherein segmentation may be performed manually or automatically, for instance by means of a segmenting neural network, and
- instead of or in addition to generating and storing a single, global coordinate system transformation during the patient registration process, generate and store an individual coordinate system transformation for each localizer or vertebra body, respectively.

This allows to reflect intraoperative movement of the predefined areas of the image data e.g., after cage placement by applying the individual coordinate system transformations of the individual body members, for instance the individual vertebra bodies.

In particular, a method is proposed that comprises the steps of:
- generating a 3D model of a mammal form preoperatively obtained tomographic image data, segmenting the 3D model for obtaining multiple 3D model segments wherein at least some 3D model segments represent rigid body parts, in particular integral bone parts such as individual vertebra bodies or bone fragments, that may move with respect to each other,
- generating individual coordinate system transformations using a navigation system for the image guided navigation by way of registration for at least some of the 3D model segments
- intraoperatively tracking the position and orientation of a plurality of individual body parts of the mammal by means of the navigation system,
- adapting a visualization of the 3D model in accordance with the respectively tracked positions and orientations of the individual body parts so as to visualize the individual body parts with their actual position and orientation relative to each other in the visualization of the 3D model,
   - wherein the adaptation of the visualization of the 3D model comprises applying the individual coordinate system transformations to the respective tracked positions and orientations of the individual body parts, and
- displaying the adapted visualization of the 3D model.

An adapted visualization of the 3D model may be generated using a generative neural network that is trained to generate 3D models.

In some embodiments, the navigation system is an electromagnetic navigation system.

According to one non-limiting embodiment, the generation of individual coordinate system transformations using a navigation system for the image guided navigation by way of registration comprises intraoperative recording of image data by way of intraoperative 3D imaging or fluoroscopy imaging.

Instead of or in addition to using a navigation system for the image guided navigation, the generation of individual coordinate system transformations may comprise
- a segmentation of the intraoperatively recorded images as represented by the intraoperatively recorded image data and
- performing registration between 3D model segments of the 3D model and corresponding segments of the intraoperatively recorded images based on the image data.

The method, in some embodiments, further comprises attaching patient localizers to a plurality of rigid body parts, in particular to a plurality of vertebra bodies. The patient localizers are, for example, Kirschner-wires or Jamshidi-needles that each comprise a localizer coil.

For automatically segmenting the 3D model or the intraoperatively obtained images, a segmenting neural network may be used that is trained wit image data representing individual rigid body parts, for instance vertebra bodies.

Further aspects of the invention will become apparent from the following description of some of the embodiments of the invention referring to the figures.

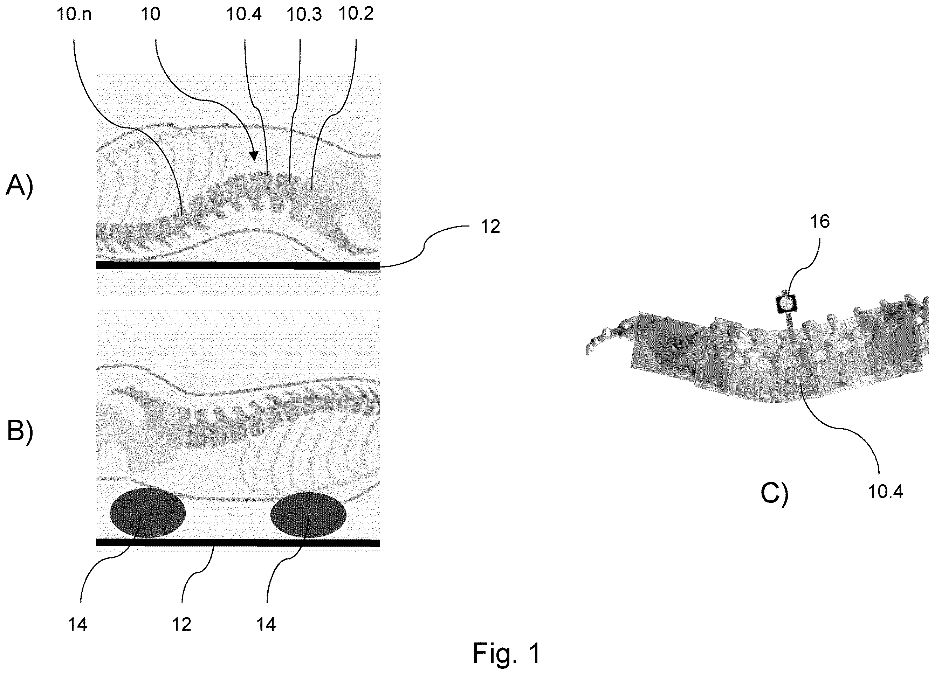

Figure 1A illustrates the shape of a spine 10 when a patient is place in a supine posture on a flat table 12. This posture may be used for obtaining preoperative image data used for generating a 3D Model.

Figure 1B illustrates the shape of the spine 10 when the patient is placed on cushions 14 and thus has a prone posture that is typical for a posture during surgery, i. e. an intraoperative posture.

When comparing figure 1A and figure 1B it is apparent that the shape of the spine 10 depends to a large degree on the posture of the patient and may vary between a preoperative state used for obtaining preoperative image data and an intraoperative state.

Figure 1C illustrates a spine 10 with individual vertebra bodies 10.1., 10.2 ... 10.n. One vertebra body 10.4 is equipped with a patient localizer 16. A correct registration between the actual position of the vertebra body 10.4 and a 3D model derived from the preoperatively obtained image data is only given for the particular vertebra body 10.4. Fig. 1C further illustrates how the accuracy of the registration decreases for vertebra bodies 10.n depending on their distance from the vertebra body 10.4 that is equipped with the patient localizer 16.

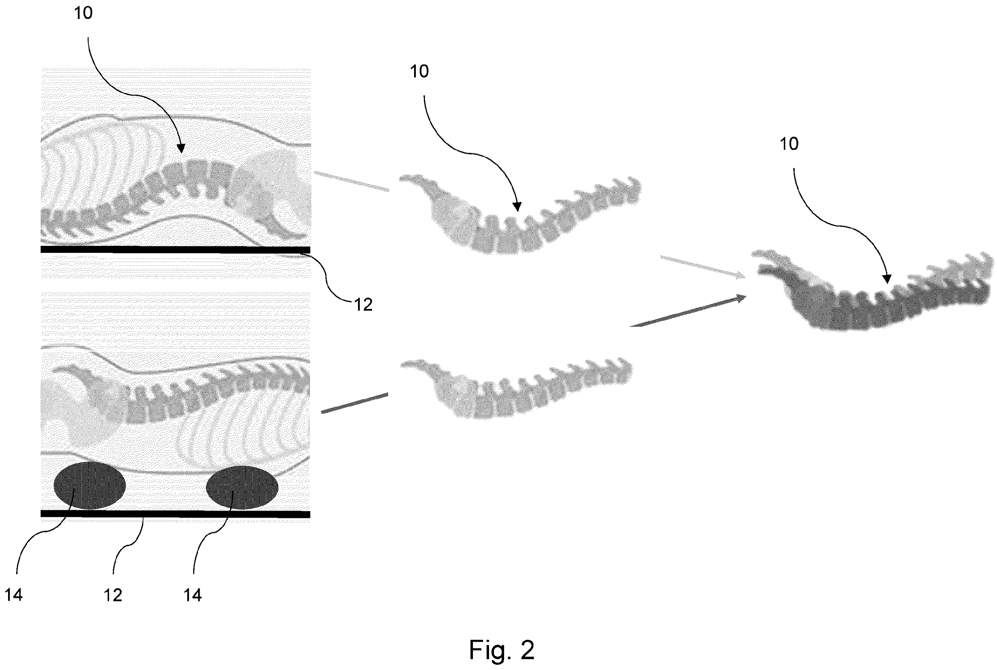

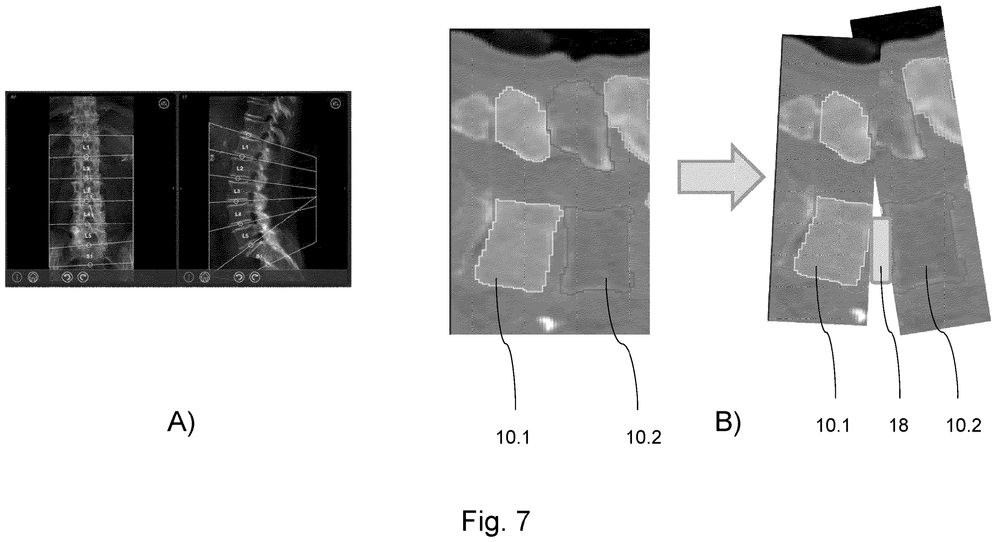

Figure 2 similarly illustrates the mismatch due to different shapes of the spine 10 before and during an operation. It is noted, that during an operation, the relative position and orientation of vertebra bodies may be changed by surgery, for instance if a cage 18 is placed between two vertebra bodies as illustrated in figure 7B.

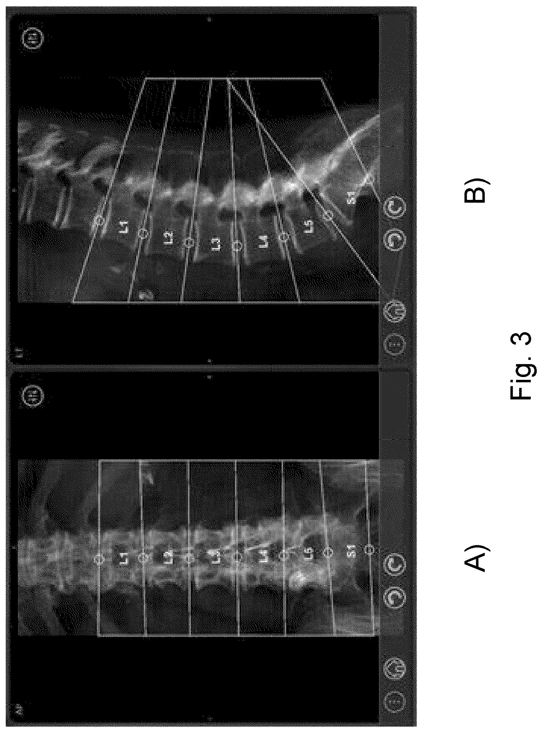

Figures 3A and B are showing two different fluoroscopic images that are intraoperatively obtained wherein the two images are segmented such that each segment of the fluoroscopic image represents a single, individual vertebra body. Segmentation of intraoperatively obtained images may be performed manually. Alternatively, a segmenting neural network may be used for automatic segmentation of the images.

The segmenting neural network may be trained with image data representing fluoroscopic images of individual vertebra bodies.

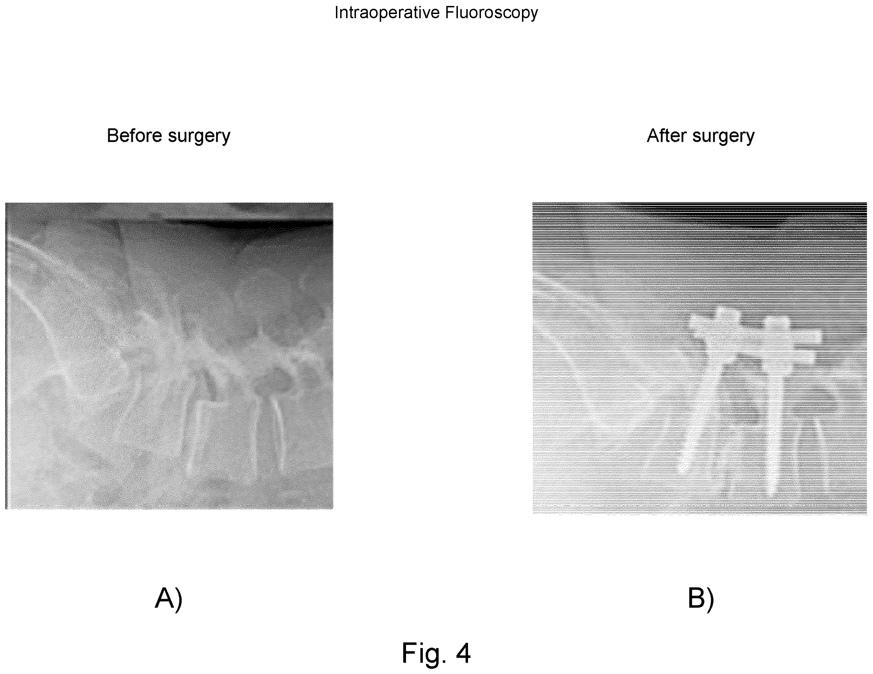

Figures 4A and B show intraoperatively obtained fluoroscopic images of vertebra bodies. Figure 4A is a fluoroscopic image that is obtained prior to surgery while figure 4B is fluoroscopic image obtained after surgery. It is apparent, that vertebra bodies 10.1 and 10.2 have changed their relative position.

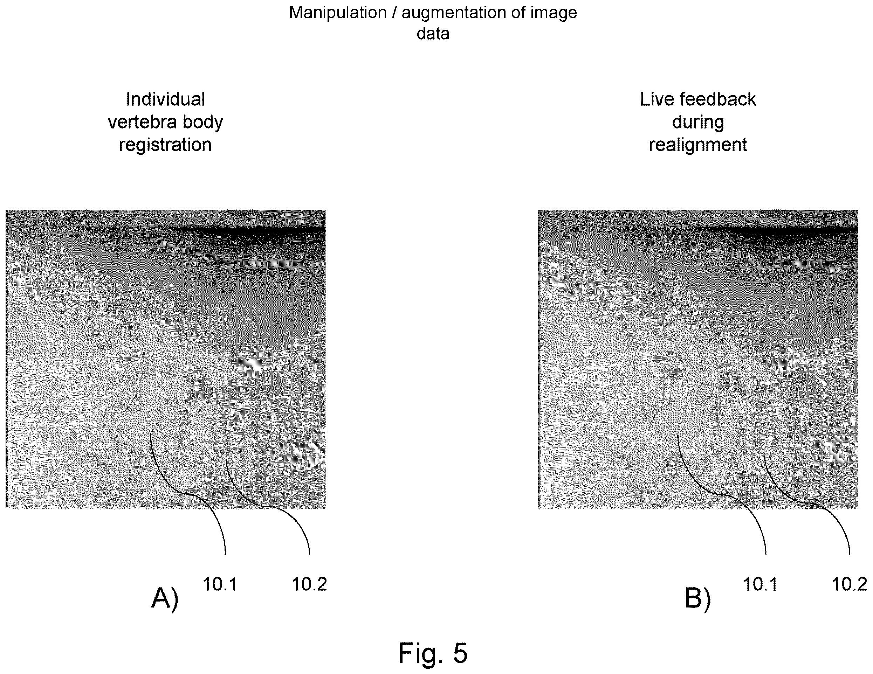

Figures 5A and B illustrate the effect of individual vertebra body registration due to image segmentation, vertebra bodies 10.1 and 10.2 are individually recognized and thus can be tracked intraoperatively. This can be used to generate augmented images as shown in figures 5A and B that reflect the actual position of individual vertebra bodies 10.1 and 10.2 before and after relative movement of the vertebra bodies 10.1 and 10.2.

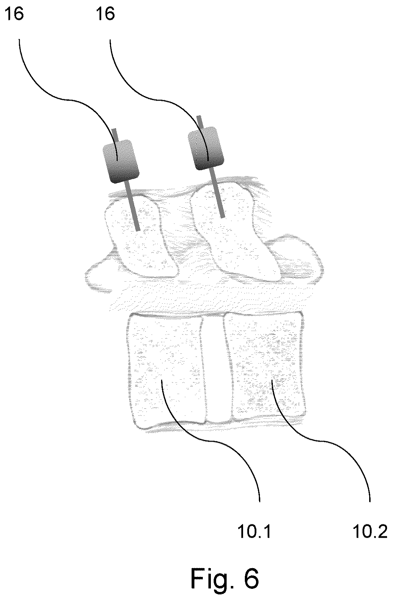

In order to allow a live tracking of the individual vertebra bodies, each vertebra body may be equipped with an individual patient localizer 16; see figure 6. According to one non-limiting embodiment, the patient localizers are navigated Kirschner-wires that each comprise at least one localizer coil.

The individual registration of rigid body parts, in particular the individual registration of individual vertebra bodies allows to reflect intraoperative movement of pre-defined areas of the image data during surgery. This is illustrated in figure 7B. Figure 7B further illustrates the image segmentation that results in individually recognized vertebra bodies 10.3 and 10.4. Figure 7A corresponds to figure 3 and illustrates how image segmentation may be performed on intraoperatively obtained fluoroscopic images.

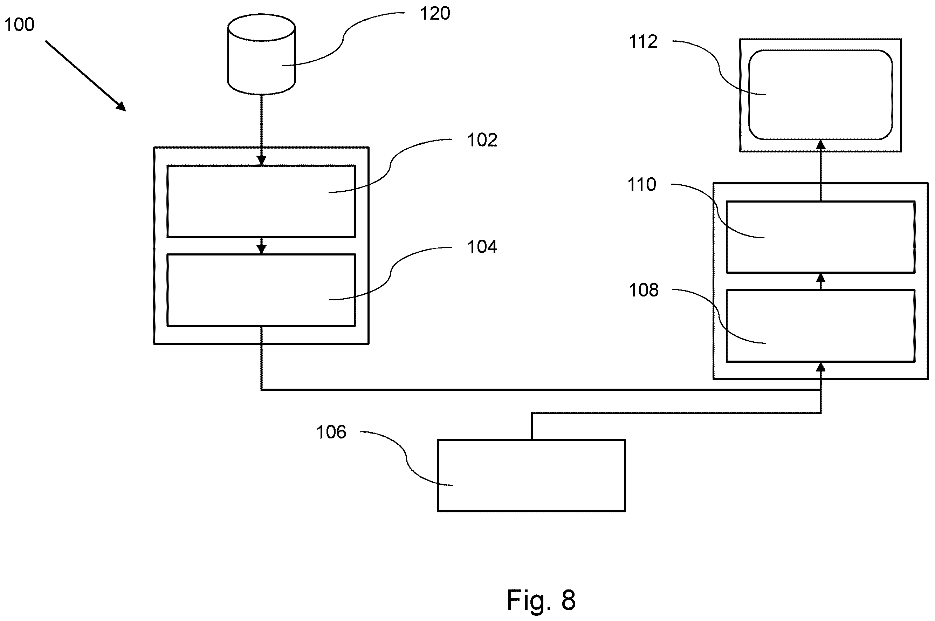

Figure 8 illustrates a system 100 for image guided navigation. The system is connected to a data storage 120 that comprises preoperatively obtained tomographic image data.

The system comprises a model generating unit 102 that is configured for generating a 3D model of a mammal form the preoperatively obtained tomographic image data. The model generating unit 102 may be part of an electro-magnetic navigation system.

The system further comprises a segmentation unit 104 that is configured for segmenting the 3D model for obtaining multiple 3D model segments wherein at least some 3D model segments represent rigid body parts, in particular integral bone parts such as individual vertebra bodies or bone fragments that may move with respect to each other.

The system further comprises a coordinate transformation unit 106 that is configured for generating individual coordinate system transformations using a navigation system for the image guided navigation by way of registration for at least some of the 3D model segments. The coordinate transformation unit 106 may be part of an electro-magnetic navigation system and provides coordinates of localized body parts in the coordinate system of the 3D model.

The system further comprises a tracking unit 108 that is configured for intraoperatively tracking the position and orientation of a plurality of individual body parts of the mammal by means of the navigation system.

The system further comprises a visualization unit 110 that is configured for adapting a visualization of the 3D model in accordance with the respectively tracked positions and orientations of the individual body parts so as to visualize the individual body parts with their actual position and orientation relative to each other in the visualization of the 3D model. The adaptation of the visualization of the 3D model comprises applying the individual coordinate system transformations to the respective tracked positions and orientations of the individual body parts.

The system further comprises a display 112 that connected to the visualization unit and that is configured for displaying the adapted visualization of the 3D model.

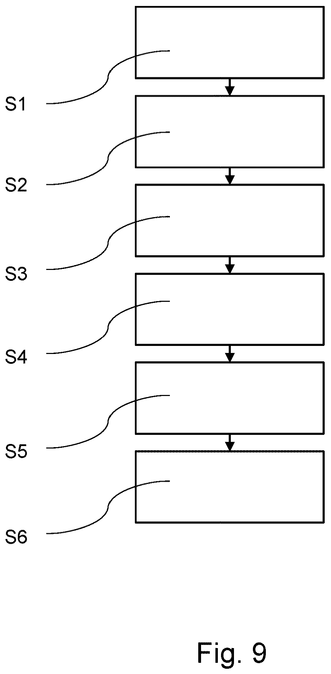

A method of operating the system comprises the steps (see figure 9):
S1: generating a 3D model of a mammal form preoperatively obtained tomographic image data by means of the model generating unit 102,
S2: segmenting - by the segmenting unit 104 - the 3D model for obtaining multiple 3D model segments wherein at least some 3D model segments represent rigid body parts, in particular integral bone parts such as individual vertebra bodies or bone fragments, that may move with respect to each other,
S3: generating - by the coordinate transformation unit 106 - individual coordinate system transformations using a navigation system for the image guided navigation by way of registration for at least some of the 3D model segments,
S4: intraoperatively tracking the position and orientation of a plurality of individual body parts of the mammal by means of the navigation system by means of the tracking unit 108,
S5: adapting - by the visualization unit 110 - a visualization of the 3D model in accordance with the respectively tracked positions and orientations of the individual body parts so as to visualize the individual body parts with their actual position and orientation relative to each other in the visualization of the 3D model, wherein the adaptation of the visualization of the 3D model comprises applying the individual coordinate system transformations to the respective tracked positions and orientations of the individual body parts, and
S6: displaying - by the display 112 - the adapted visualization of the 3D model.

### Reference numerals:

- 10: spine
- 10.1, 10.2 ... 10.n: vertebra body
- 12: table
- 14: cushion
- 16: patient localizer
- 18: cage
- 100: system
- 102: model generating unit
- 104: segmentation unit
- 106: coordinate transformation unit
- 108: tracking unit
- 110: visualization unit
- 112: display
- 120: data storage

## Claims

1. A method of image guided navigation, comprising the steps of
- generating a 3D model of a mammal form preoperatively obtained tomographic image data,
- segmenting the 3D model for obtaining multiple 3D model segments wherein at least some 3D model segments represent rigid body parts, in particular integral bone parts such as individual vertebra bodies or bone fragments, that may move with respect to each other,
- generating individual coordinate system transformations using a navigation system for the image guided navigation by way of registration for at least some of the 3D model segments
- intraoperatively tracking the position and orientation of a plurality of individual body parts of the mammal by means of the navigation system,
- adapting a visualization of the 3D model in accordance with the respectively tracked positions and orientations of the individual body parts so as to visualize the individual body parts with their actual position and orientation relative to each other in the visualization of the 3D model,
- wherein the adaptation of the visualization of the 3D model comprises applying the individual coordinate system transformations to the respective tracked positions and orientations of the individual body parts, and
- displaying the adapted visualization of the 3D model.

2. The method of claim 1, wherein the navigation system is an electromagnetic navigation system.

3. The method of claim 1 or 2, wherein the generation of individual coordinate system transformations using a navigation system for the image guided navigation by way of registration comprises intraoperative recording of image data by way of intraoperative 3D imaging or fluoroscopy imaging.

4. The method of claim 3, comprising segmentation of the intraoperatively recorded images as represented by the intraoperatively recorded image data and performing registration between 3D model segments of the 3D model and corresponding segments of the intraoperatively recorded images.

5. The method of at least one of claims 1 to 4, further comprising attaching patient localizers to a plurality of rigid body parts, in particular to a plurality of vertebra bodies.

6. The method of claim 5, wherein the patient localizers are Kirschner-wires or Jamshidi-needles that each comprise a localizer coil.

7. System (100) for image guided navigation, comprising
- a model generating unit (102) that is configured for generating a 3D model of a mammal form preoperatively obtained tomographic image data,
- a segmentation unit (104) that is configured for segmenting the 3D model for obtaining multiple 3D model segments wherein at least some 3D model segments represent rigid body parts, in particular integral bone parts such as individual vertebra bodies or bone fragments, that may move with respect to each other,
- a coordinate transformation unit (106) that is configured for generating individual coordinate system transformations using a navigation system for the image guided navigation by way of registration for at least some of the 3D model segments
- a tracking unit (108) that is configured for intraoperatively tracking the position and orientation of a plurality of individual body parts of the mammal by means of the navigation system,
- a visualization unit (110) that is configured for adapting a visualization of the 3D model in accordance with the respectively tracked positions and orientations of the individual body parts so as to visualize the individual body parts with their actual position and orientation relative to each other in the visualization of the 3D model,
- wherein the adaptation of the visualization of the 3D model comprises applying the individual coordinate system transformations to the respective tracked positions and orientations of the individual body parts, and
- a display (112) being connected to the visualization unit and being configured for displaying the adapted visualization of the 3D model.

8. Method of operating the system (100) of claim 8, comprising the steps of:
- generating a 3D model of a mammal form preoperatively obtained tomographic image data by means of the model generating unit (10)2,
- segmenting - by the segmenting unit (104) - the 3D model for obtaining multiple 3D model segments wherein at least some 3D model segments represent rigid body parts, in particular integral bone parts such as individual vertebra bodies or bone fragments, that may move with respect to each other,
- generating - by the coordinate transformation unit (106) - individual coordinate system transformations using a navigation system for the image guided navigation by way of registration for at least some of the 3D model segments,
- intraoperatively tracking the position and orientation of a plurality of individual body parts of the mammal by means of the navigation system by means of the tracking unit (108),
- adapting - by the visualization unit (110) - a visualization of the 3D model in accordance with the respectively tracked positions and orientations of the individual body parts so as to visualize the individual body parts with their actual position and orientation relative to each other in the visualization of the 3D model, wherein the adaptation of the visualization of the 3D model comprises applying the individual coordinate system transformations to the respective tracked positions and orientations of the individual body parts, and
- displaying - by the display (112) - the adapted visualization of the 3D model.
